Europäisches Patentamt

(19) European Patent Office

Office européen des brevets

(11) Publication number: **0 143 618**
**B1**

# (12) EUROPEAN PATENT SPECIFICATION

(45) Date of publication of patent specification: **13.07.88**

(51) Int. Cl.⁴: **A 61 M 16/20**

(21) Application number: **84308116.7**

(22) Date of filing: **22.11.84**

(54) **Exhalation valve.**

(30) Priority: **25.11.83 US 554877**

(43) Date of publication of application:
**05.06.85 Bulletin 85/23**

(45) Publication of the grant of the patent:
**13.07.88 Bulletin 88/28**

(84) Designated Contracting States:
**DE FR GB SE**

(56) References cited:
**DE-A-3 209 643**
**GB-A- 826 280**
**US-A-2 121 549**
**US-A-4 241 756**

(73) Proprietor: **The BOC Group, Inc.**
**85 Chestnut Ridge Road**
**Montvale, New Jersey 07645 (US)**

(72) Inventor: **Schroeder, Gerhardt P.**
**5315 Barton Road**
**Madison Wisconsin 53711 (US)**

(74) Representative: **Gough, Peter et al**
**c/o THE BOC GROUP PLC Patent Department**
**Chertsey Road**
**Windlesham Surrey GU20 6HJ (GB)**

## Description

The present invention relates to an exhalation valve for use in a respiratory breathing circuit for controlling the exhalation of a patient utilizing that breathing circuit and to control the minimum pressure within that patient circuit.

There are presently various forms of exhalation valves for respiratory breathing circuits. One fairly popular commercial type is the mushroom valve wherein an inflatable bladder is located adjacent an opening through which the patient's exhaled breath passes. By inflating the bladder, that bladder expands to the point where it covers and thus seals the opening and prevents the passage of gas therethrough. One difficulty with such a valve is, however, that it may materially deform after prolonged use and fail to provide an adequate seal.

A further type of exhalation valve, as shown in U.S. Patent 4,241,756 includes a flexible diaphragm that covers the opening and a sealed chamber above the diaphragm is selectively pressurized to create a bias urging against the diaphragm to close the same or to retain a bias against that diaphragm urging it toward its closed position.

A difficulty with that flexible diaphragm type of valve is, however, that the flexibility of the diaphragm causes instability thereof, i.e. the diaphragm may not move uniformly from its seat but may flex only in a certain area thereof and thus release pressure in the patient circuit at a level unanticipated by the control pressure in the sealed chamber.

If, on the other hand, the diaphragm is comprised of a rigid material to overcome the flexing problem, the rigid diaphragm does not allow good sealing against the valve opening and leakage is a further difficulty.

According to the present invention, an exhalation valve comprises a valve housing forming an inlet and an outlet, a valve seat formed in said housing, and a flexible diaphragm adapted to have one face thereof move against and away from said valve seat to, respectively, close and open said exhalation valve, said housing further forming a closed control chamber acting upon the other face of said flexible diaphragm, and port means in said housing for effecting a control pressure to said closed control chamber for controlling the movement of said flexible diaphragm, in which said flexible diaphragm has a fairly rigid plunger comprising a disc and a rigid projection extending from the disc, said disc being fitted into said flexible diaphragm and the projection being adapted to move upon movement of the flexible diaphragm within and be constrained by a bore in said valve housing, said rigid projection and said bore being constructed to restrain movement of said flexible diaphragm to a stable path with respect to said valve seat (38) substantially along the main axis of said projection.

Preferably, the improved diaphragm is utilized having a resilient outer peripheral portion and which surrounds an inner portion or plunger of fairly rigid material and having a damping means to control and stabilize the movement of the diaphragm. As a part of the plunger, a projection extends outwardly therefrom and moves, throughout its stroke, within a suitable shaped cylindrical bore and which serves to align the movement of the diaphragm itself as well as damp its motion.

In a preferred embodiment, the plunger is fitted into the outer peripheral portion for ease in moulding and assembly. Thus the outer peripheral portion of the diaphragm is flexible and thus effects a good seal against the valve opening and yet the registered plunger provides stability of movement of the overall diaphragm.

The valve itself is relatively simple to manufacture in that the diaphragm is assembled by snapping the plunger into the inner opening of the outer peripheral portion and the diaphragm thus placed over the inlet for gases exhaled from the patient. A cap is snapped over the flexible diaphragm to enclosed the same and to form the enclosed chamber which is pressurized to both move the diaphragm to the closed position and to maintain a desired PEEP level of pressure within the patient circuit. The cylindrical bore is in alignment with and receives the projection extending from the plunger.

Thus the unique diaphragm having a fairly rigid plunger with a projection extending therefrom and a flexible outer peripheral portion is stabilized in its movement between open and closed positions and yet which provides a good seal about the valve opening.

An embodiment of the invention will now be described, by way of example, reference being made to the Figures of the accompanying diagrammatic drawings in which:—

Fig. 1 is a schematic view of a respiratory breathing circuit containing an exhalation valve constructed in accordance with the present invention; and

Fig. 2 is a cross-sectional view of the exhalation valve usable with the circuit of Fig. 1.

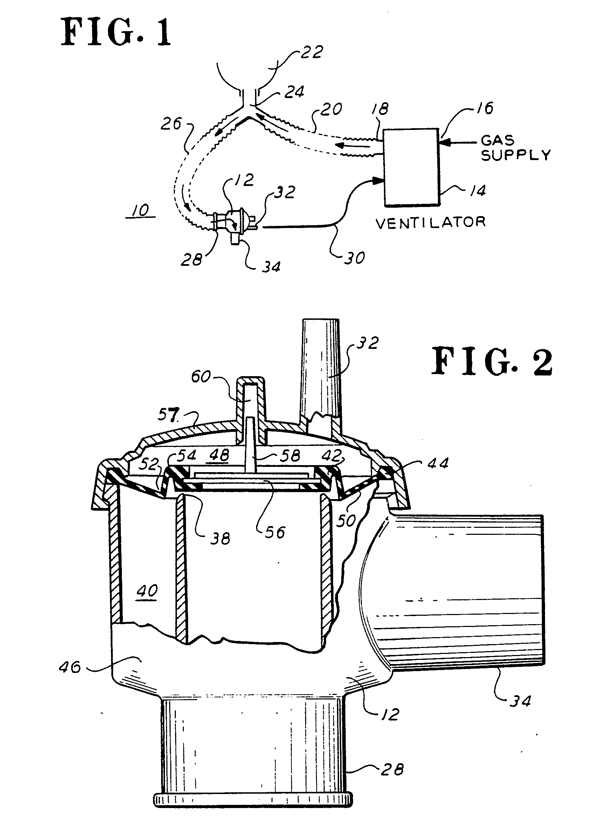

In Fig. 1 there is shown a schematic view of a respiratory breathing circuit 10 in which the exhalation valve 12 of the present invention is utilized. As herein described, exhalation valve 12 will be specifically utilized in an open respiratory breathing circuit, however, the exhalation valve 12 may also be readily used in a closed anesthesia circuit.

In the respiratory breathing circuit 10, as shown, the normal components include a ventilator 14 that supplies the gas to the patients lungs and which also provides a pressure signal for operation of the exhalation valve 12. A supply of gas is made availabe to the ventilator 14 as is well known in the art via an inlet 16. An outlet 18 from the ventilator 14 is connected to a flexible tubing 20 that carries gas from ventilator 14 to some means of communicating that gas to a patient's lungs. A patient mask 22 is shown which is connectible to the flexible tubing 20 by a "Y" connector 24. A further flexible tubing 26 carries gas from the patient mask 22 and also is connected to "Y" connector 24.

That flexible tubing 26 carrying exhalation gas from the patient connects to the exhalation valve inlet 28. A small tubing 30 communicates between the ventilator 14 and a port 32 in exhalation valve 12, the purpose of which will be later explained. The small tubing 30 does, however, communicate a pressure signal from ventilator 14 to exhalation valve 12 to control gas passing through exhalation valve 12 between the exhalation valve inlet 28 and exhalation valve outlet 34.

Turning now to Fig. 2, there is shown a cross-sectional view of the exhalation valve 12 constructed for use in a breathing circuit such as used in Fig. 1, in accordance with this invention. The valve 12 includes a valve housing 46 having inlet 28 and outlet 34.

The inlet 28 is formed within valve housing 46, as shown, as a cylinder which continues upwardly within the housing 46 and forms a valve seat 38 at the top thereof as a round opening.

An annular exhaust chamber 40 surrounds the inlet 28 and communicates directly with the outlet 34.

A flexible diaphragm 42 is positioned atop the valve seat 38 and cooperates therewith to open or close the exhalation valve 12. At the outer peripheral edge of flexible diaphragm 42 there is formed a bead 44 that is sealed between valve housing 46 and a cap 57. The cap 57 is assembled to the valve housing 46 by a snap fit.

A control chamber 48 is thus formed between the cap 57 and the flexible diaphragm 42 and port 32 is formed in cap 57 through which an outside source of pressure can communicate to control the pressure within control chamber 48.

Flexible diaphragm 42 is comprised of a flexible outer peripheral portion 50 having flexible hinges 52 and 54 to enable movement of the flexible diaphragm 42 with minimum resistance. In the centre of flexible diaphragm 42, there is a fairly rigid plunger 56. In a preferred embodiment, the plunger 56 merely snaps into a suitable shaped opening that is moulded in the flexible outer peripheral portion 50. The fairly rigid plunger 56 may be constructed of polypropylene while the flexible outer peripheral portion 50 may be of silicone but other flexible materials can be utilized that are preferably sterilizable by autoclave or ethylene oxide.

Extending upwardly from plunger 56 is a projection 58 that rides within cylindrical bore 60 formed in the cap 57.

In the operation of the exhalation valve 12, gases expired from the patient pass into the exhalation valve 12 through the inlet 28. During the time the ventilator is in the inspiration phase, that is, gas is being delivered to the patient by the ventilator, a sufficient control pressure is communicated from ventilator 14 to the control chamber 48 through port 32 to create a force against the flexible diaphragm 42 retaining it in the closed position. In the closed position, not shown, the flexible outer peripheral portion 50 of the flexible diaphragm 42 seals against the valve seat 38 to prevent the passage of gas therethrough, that is, there is no means of communication between the inlet 28 and the outlet 34 of the exhalation valve 12.

As the ventilator ends the inhalation phase and the patient exhales, the pressure communicated through the port 32 by ventilator 14 to control chamber 48 is decreased so that the flexible diaphragm 42 an be raised by the flow of exhalation gas from the patient. Such gas may pass through the exhalation valve 12 from inlet 28 to outlet 34. Opening of the flexible diaphragm 42 is controlled, such as to maintain PEEP, by controlling the pressure, during exhalation, within the control chamber 48 by the ventilator 14.

During opening and closing of exhalation valve 12, the flexible diaphragm 42 is readily moveable by means of flexible hinges 52 and 54 yet retains stability through rigid plunger 56 having its projection 58 retained within cylindrical bore 60 in cap 57, thus the flexible diaphragm 42 moves up and down in a set path and the movement of flexible diaphragm 42 moves fairly uniformly with respect to valve seat 38 so that opening and closing of exhalation valve 12 is stable, yet good sealing is achieved in the closed position.

While the present invention has been particularly set forth in terms of specific embodiments thereof, it will be understood in view of the instant disclosure, that numerous variations upon the invention are now enabled to those skilled in that art, which variations yet reside within the scope of the instant teaching.

**Claims**

1. An exhalation valve comprising a valve housing (46) forming an inlet (28) and an outlet (34), a valve seat (38) formed in said housing (46), and a flexible diaphragm (42) adapted to have one face thereof move against and away from said valve seat (38) to, respectively, close and open said exhalation valve, said housing (46) further forming a closed control chamber (48) acting upon the other face of said flexible diaphragm (42), and port means (32) in said housing (46) for effecting a control pressure to said closed control chamber (48) for controlling the movement of said flexible diaphragm (42), characterized by said flexible diaphragm (42) having a fairly rigid plunger (56) comprising a disc and a rigid projection (58) extending from the disc, said disc being fitted into said flexible diaphragm (42) and the projection (58) being adapted to move upon movement of the flexible diaphragm (42) within and be constrained by a bore (60) in said valve housing (46), said rigid projection (58) and said bore (60) being constructed to restrain movement of said flexible diaphragm (42) to a stable path with respect to said valve seat (38) substantially along the main axis of said projection (58).

2. An exhalation valve as claimed in claim 1, characterised in that said housing (46) comprises a main body and a cap (57) fitted thereto and said bore (60) comprises a recess formed in said cap (57).

3. An exhalation valve as claimed in claim 2, characterised in that said flexible diaphragm (42) is retained in position by its periphery held between said cap (57) and said main body.

4. An exhalation valve as claimed in claim 2, characterised in that said flexible diaphragm (42) has at least a pair of moulded hinges (52, 54) to provide flexibility.

**Patentansprüche**

1. Ausatmungsventil mit einem Ventilgehäuse (46) das einen Einlaß (28) und einen Auslaß (34) bildet, einem in dem Gehäuse (46) ausgebildeten Ventilsitz (38) und einer flexiblen Membran (42), die so ausgelegt ist, daß eine Fläche derselben sich zu dem Ventilsitz (38) hin bzw. von diesem weg bewegen kann und das Ausatemventil schließt bzw. öffnet, wobei das Gehäuse (46) weiterhin eine geschlossene Steuerkammer (48) bildet, die auf die andere Fläche der flexiblen Membran (42) einwirkt, und mit Anschlußmitteln (32) in dem Gehäuse (46), um einen Steuerdruck für die geschlossene Steuerkammer (48) zu bewirken, um die Bewegung der flexiblen Membran (42) zu steuern, dadurch gekennzeichnet, daß die flexible Membran (42) einen ziemlich starren Stößel (56) besitzt mit einer Scheibe und einem starren, von der Scheibe abstehenden Fortsatz (58), wobei die Scheibe in die flexible Membran (42) eingepaßt ist und der Fortsatz (58) dazu ausgelegt ist, sich bei einer Bewegung der flexiblen Membran (42) in einer Bohrung (60) in dem Ventilgehäuse (46) zu bewegen und in ihr gehalten zu sein, wobei der starre Fortsatz (58) und die Bohrung (60) aufgebaut sind, eine Bewegung der flexiblen Membran (42) auf einen stabilen Weg bezüglich des Ventilsitzes (38) im wesentlichen längs der Hauptachse des Fortsatzes (58) zu beschränken.

2. Ausatmungsventil nach Anspruch 1, dadurch gekennzeichnet, daß das Gehäuse (46) einen Hauptkörper und einen darauf gepaßten Deckel (57) umfaßt und daß die Bohrung (60) einen in dem Deckel (57) ausgebildeten Einschnitt umfaßt.

3. Ausatmungsventil nach Anspruch 2, dadurch gekennzeichnet, daß die flexible Membran (42) in ihrer Lage dadurch zurückgehalten wird, daß ihr Umfang zwischen dem Deckel (57) und dem Hauptkörper gehalten ist.

4. Ausatmungsventil nach Anspruch 2, dadurch gekennziechnet, daß die flexible Membran (42) mindestens ein Paar geformte Gelenklinien (52, 54) zur Schaffung von Flexibilität besitzt.

**Revendications**

1. Valve d'expiration comprenant un corps (46) qui forme un passage d'entrée (28) et un passage de sortie (34), un siège (38) formé dans ce corps (46), un diaphragme flexible (42) agencé de manière qu'une de ses faces se rapproche et s'éloigne de ce siège (38) en, respectivement, fermant et ouvrant la valve (12), le corps (46) formant par ailleurs une chambre (48) hermétique de réglage agissant sur l'autre face de ce diaphragme (42), et un passage (32) formé dans ledit corps (46) de façon à créer une pression de réglage dans cette chambre (48), de manière à régler le déplacement du diaphragme flexible (42), valve caractérisée en ce que ce diaphragme flexible (42) comporte un plongeur (56) assez rigide, comprenant un disque et un prolongement (58) rigide partant de ce disque, celui-ci étant ajusté dans le diaphragme (42) et le prolongement (58) étant destiné à se déplacer, quand ledit diaphragme se déplace, et à être retenu dans un tube borgne (60) ménagé dans le corps (46), ledit prolongement (58) et ce tube (60) étant conformée de manière à limiter le déplacement du diaphragme (42) à un trajet stable par rapport au siège (38) et suivant pratiquement l'axe de ce prolongement.

2. Valve d'expiration selon la revendication 1, caractérisée en ce que le corps (46) comprend un corps proprement dit et un couvercle (57), ajusté sur lui, le tube borgne (60) comprenant un évidement formé dans ce convercle (57).

3. Valve d'expiration selon la revendication 2, caractérisée en ce que le diaphragme flexible (42) est retenu en place par sa périphérie extérieure maintenue entre le couvercle (57) et le corps proprement dit.

4. Valve d'expiration selon la revendication 2, caractérisée en ce que le diaphragme flexible (42) comporte au moins deux charnières moulées (52, 54) destinées à lui donner une certaine flexibilité.

0 143 618

# FIG. 1

# FIG. 2

1